# EUROPÄISCHE PATENTSCHRIFT

(11) **EP 3 361 230 B1**
(45) Veröffentlichungstag und Bekanntmachung des Hinweises auf die Patenterteilung: **29.03.2023**
(21) Anmeldenummer: 18155874.3
(22) Anmeldetag: 08.02.2018
(51) Int. Cl.: G01N 13/00, G01N 21/05, G01N 33/15, G01N 1/38, G01N 35/00

(54) **DURCHFLUSSZELLE FÜR EIN DISSOLUTION TESTGERÄT**
FLOW CELL FOR A DISSOLUTION TEST DEVICE
CELLULE D'ÉCOULEMENT POUR UN APPAREIL D'ESSAI DE DISSOLUTION

(30) Priorität: 13.02.2017 DE 102017102761
(43) Veröffentlichungstag der Anmeldung: 15.08.2018
(73) Patentinhaber: Erweka GmbH, 63225 Langen (DE)
(72) Erfinder: Müller, Werner, 63150 Heusenstamm (DE); Bozkurt, Levent, 61191 Rosbach (DE)
(74) Vertreter: Meyer-Dulheuer MD Legal Patentanwälte PartG mbB

(56) Entgegenhaltungen:
- WO-A1-94/22566
- DE-A1- 4 037 720
- DE-T2- 69 017 166
- US-A- 4 578 244
- US-A- 5 127 278
- US-A1- 2010 202 929

## Beschreibung

Gegenstand der vorliegenden Erfindung sind eine Durchflusszelle für ein Dissolution Testgerät, ein entsprechendes Dissolution Testgerät und ein Verfahren zur Prüfdurchführung mit einem Dissolution Testgerät.

Zur Prüfung der Eigenschaften von pulver- und granulatförmigen Medikamenten, Tabletten, Suppositorien, Implantaten und Stents gehört auch die Prüfung ihrer Auflösungseigenschaften. Dazu werden die Prüflinge in Dissolution Testgeräten in eine Durchflusszelle verbracht, dort mit einem Prüfmedium bei einer definierten Temperatur umspült und dann die Menge an freigesetztem Wirkstoff oder aufgelöstem Prüfling in dem Prüfmedium in Abhängigkeit von der Zeit erfasst.

Im Stand der Technik wird der Begriff "Durchflusszelle" oftmals alleine für die in dieser Anmeldung als "Probenzelle" bezeichnete Zelle, die die zu untersuchende Probe aufnimmt und vom Prüfmedium durchströmt wird, verwendet. Manchmal wird er auch unscharf sowohl für die Probenzelle als auch für die gesamte Zelle mit dem die Prüfzelle umgebenden Zellenmantel und Filterkopf verwendet. Im Sinne dieser Anmeldung ist unter dem Begriff "Durchflusszelle" jedoch stets der gesamte Aufbau einer Messzelle mit der Zellenaufnahme, dem Zellenmantel, der Probenzelle und dem Filterkopf zu verstehen.

Die zu verwendenden Durchflusszellen sind, was ihre apparativen Parameter und ihre Betriebsweise anbelangt, weitgehend in den nationalen und internationalen Arzneibüchern normiert bzw. standardisiert. Beispielhaft seien hier die Europäische Pharmacopoeia 5.0, Kapitel 2.9.3. "Dissolution test for solid dosage forms", Seiten 228 bis 230, von 2005 und die United States Pharmacopeial Convention (USP) 2011, Kapitel 711 "Dissolution", Seiten 1 bis 8, genannt. Die Dissolution Testgeräte bestehen aus einem Wasserbad, in dem sich eine Heizspirale für das Prüfmedium befindet und auf das mittels der Zellenaufnahme die Durchflusszelle montiert wird.

Das Prüfmedium, bei dem es sich in der Regel um künstlichen Magen- oder Darmsaft handelt, wird mit einer konstanten Flussgeschwindigkeit durch die Heizspirale in die Probenzelle gepumpt, wo es den Prüfling auflöst. Dabei wird es einerseits in der Heizspirale auf die Prüftemperatur temperiert und andererseits sorgt eine gleichzeitige Durchspülung des Zellenmantels mit dem Wasser des Wasserbads für eine Temperierung der Probenzelle von außen und damit für Temperaturkonstanz im Gesamtsystem.

Das Prüfmedium durchströmt die Probenzelle dabei über eine Einlassöffnung am Zellenboden in Richtung Auslassöffnung im Kopf der Zelle. Dazwischen durchfließt es verschiedene Elemente, die die Strömung beeinflussen (z. B. Glaskugeln), die jeweiligen Proben bzw. Formulierungen halten und Rückstände ausfiltern. Die Bestückung der Probenzelle mit Art und Anzahl der Elemente und der Proben richtet sich nach den Testmethoden, die ausgeführt werden. Dementsprechend werden auch die jeweils passenden Zellenköpfe montiert. Das oberste Element der Probenzelle vor dem Auslass bildet immer ein Filter, der ungelöste Partikel in der Zelle zurückhält. Nach Verlassen der Probenzelle durch die Auslassöffnung im Kopf wird das Prüfmedium dann einem Probensammler oder einem online Messgerät (z. B. UV-Messgerät) zugeführt.

Da die Durchflusszelle und die Probenzelle sich zum Reinigen und zur Bestückung mit den Prüflingen demontieren lassen müssen, werden diese üblicherweise steckbar konstruiert. Dabei werden die einzelnen Teile durch eine elektrische oder mechanische Klemmvorrichtung, die die zusammengesteckten Teile von oben auf das Wasserbad bzw. die darin befestigte Zellenaufnahme drückt, während der Prüfung zusammengehalten. Die Abdichtung erfolgt dabei durch toroide O-Ring-Dichtungen, welche entweder in Rillen am äußeren Umfang der Teile eingelegt sind und so gleichzeitig eine Führung und provisorische Halterung der Teile ermöglichen oder zwischen Kopf und Filter eingelegt werden. In letzterem Fall ist die Unterseite des Kopfes dann mit einem abgeschrägten Randbereich versehen, damit die O-Ring-Dichtung nach außen gegen die Wandung gedrückt werden kann.

Bei den Filtern existieren, je nach verwendeter Probenzellenart, zwei unterschiedliche Varianten. Die erste Variante, welche überwiegend für die Messung von Granulaten, Pulvern, Stents und Implantaten verwendet wird, wird unterhalb der Dichtung platziert und setzt sich aus einem stützenden grobmaschigen Sieb und einem darauf gelegten Filter aus Papier oder Glasfaservlies zusammen. Diese legen sich dann an die Unterseite des Kopfes an, der dazu im Zentrum einen über die untere Fläche, mit der er auf dem Dichtungsring aufsitzt, hinausragenden Zylinder besitzt, der in das obere Ende des Probenraums hineinragt.

Die zweite, überwiegend für Tabletten und Cremes verwendete Variante platziert den Filter oberhalb der Dichtung und besteht aus einem Filterpaket, das in einer Aussparung in der unteren Abschlussfläche des Kopfes eingelegt wird.

Das Dokument DE3133373A1 beschreibt eine Vorrichtung zur Untersuchung von Teilchen mit einem ersten Gefäß zur Aufnahme einer elektrolytischen Flüssigkeit mit darin suspendierten Teilchen und einer Elektrolyt enthaltenden ersten Kammer, einer einen Elektrolyt enthaltenden zweiten Kammer, einer Messöffnung in der Wandung zwischen dem Gefäß und der ersten Kammer, einer Spülöffnung in der Wandung zwischen der ersten Kammer und der zweiten Kammer, einem elektrischen Stromfluss durch die Messöffnung zur Erzeugung erfassbarer Signale bei Durchtritt von Teilchen durch die Messöffnung, und mit einem Flüssigkeitskreislaufsystem, welches eine Strömung von der zweiten Kammer durch ein die Teilchen zurückhaltendes Filter in die erste Kammer erzeugt, welche durch die kinetische Energie der aus der Messöffnung abfließenden Suspension in Bewegung gehalten wird. Die darin beschriebene Durchflusszelle weist einen Gehäusekörper mit zwei Endteilen auf, welche jeweils an den entgegengesetzten Enden in den Gehäusekörper eingeschraubt sind.

Das Dokument US4181853A beschreibt eine Vorrichtung zur Flüssigkeitschromatographie, bei der eine Flüssigkeitsfraktion aus einer Chromatographiesäule durch eine Durchflussmesszelle, die mit einer stationären Phase gefüllt ist, die die zu absorbierende Spezies absorbiert, geleitet wird, wobei die zu messende Spezies an der stationären Phase in der Durchflusszelle absorbiert wird und die Messung durch die emittierte Fluoreszenz vorgenommen wird, die in der zu messenden Spezies durch die Einstrahlung elektromagnetischer Strahlung angeregt wird, und wobei die fluoreszierende Spezies bei der Messung im Gleichgewicht zwischen der stationären und der mobilen Phase in der Messzelle durch Fluoreszenz in niedrigeren Konzentrationen messbar ist als im Gleichgewicht nur mit der mobilen Phase. Die Durchflusszelle kann mit Endkappen in der Vorrichtung zur Flüssigkeitschromatographie, die mit der Durchflusszelle verschraubbar sind, verbunden werden.

Das Dokument US4578244A beschreibt eine Probenmessvorrichtung zur Aufnahme einer Vielzahl von Flüssigkeitsproben, welche zur Messung der Lösegeschwindigkeit einer Vielzahl von Proben verwendbar ist, die Vorrichtung umfassend mehrere Probeaufnahmebehälter mit jeweils einer Kammer zur Aufnahme einer Probe und einer Flüssigkeit, ein Tablett mit einer Antriebsvorrichtung zum gleichzeitigen Transport mehrerer Probeaufnahmebehälter zu einer Probeentnahmestation, Flüssigkeitszuleitungen für jeden der Probeaufnahmebehälter, um eine Testflüssigkeit in jeden der Probeaufnahmebehälter zu leiten, wenn die Probeaufnahmebehälter an der Probeentnahmestation positioniert sind, und Flüssigkeitsabflussleitungen für jeden der Probeaufnahmebehälter, um Testflüssigkeit zu entnehmen, wenn die Probeaufnahmebehälter an der Probeentnahmestation positioniert sind, und Kupplungseinrichtungen zum Verbinden der Flüssigkeitszuleitung und der Flüssigkeitsabflussleitung mit den Zu- und Ableitungskanälen der Kammer in jedem der Probeaufnahmebehälter, wenn die Probeaufnahmebehälter an der Probeentnahmestation positioniert sind, wobei die Kupplungseinrichtungen angepasst sind, um die Probeaufnahmebehälter einzeln abzudichten, wenn die Flüssigkeitszuleitung und die Flüssigkeitsabflussleitung mit den Zu- und Ableitungskanälen der Kammer der Probeaufnahmebehälter verbunden sind. Die Kupplungseinrichtungen pressen die Flüssigkeitszuleitungen und Flüssigkeitsabflussleitungen beim Kupplungsvorgang mit speziellen Antrieben gegen die Zu- und Ableitungskanäle der Kammer in jedem der Probeaufnahmebehälter, wobei gleichzeitig sprungfederbewehrte Stifte in Aufnahmevorrichtungen in dem Tablett und in den Probeaufnahmebehältern gedrückt werden, wodurch ein Verrutschen der Probeaufnahmebehälter beim Zu- und Ableiten der Flüssigkeit nicht möglich ist. Dadurch wird insgesamt eine hohe Dichtigkeit bei der Messung der Lösegeschwindigkeit erreicht.

Das Dokument DE 40 37 720 A1 offenbart eine Apparatur zur Auflösungsprüfung von festen Arzneimittelformen und den Oberbegriff des Anspruchs 1.

Bei der Abdichtung der Durchflusszellen treten im Stand der Technik jedoch immer wieder Probleme auf, die zu Undichtigkeiten zwischen dem Wasserkreislauf und dem Prüfmedium-Kreislauf führen. Auf diese Weise kann Wasser in das Prüfmedium gelangen und so für eine unerwünschte Verdünnung sorgen oder Prüfmedium kann ins Wasserbad austreten und dort für Verunreinigungen und Verlust an gelöstem Wirkstoff bzw. Prüfling sorgen.

Derlei Messfehler werden bereits durch kleinere Ungenauigkeiten bei der Fluchtung der zusammengesetzten Teile verursacht. Dabei ist es schwierig, diese Fehlerquelle im laufenden Betrieb zu erkennen, soweit nicht eine komplette Leckage der Zelle auftritt. Dies führt dazu, dass die Messungen nicht reproduzierbar sind und bei Vorschriften, bei denen mehrere Proben parallel vermessen werden, eine große Schwankung innerhalb eines Messsatzes auftritt. Außerdem bedeutet es einen erheblichen Zeitverlust für eine Messreihe, wenn Prüfmedium in den Zellenmantel austritt, da es dann nicht ausreicht, einfach nur die Probenzelle für die nächste Messung neu zu bestücken, sondern das Wasser im Wasserbad getauscht und sämtliche Durchflusszellen und das Wasserbad gereinigt werden müssen.

Die Aufgabe der vorliegenden Erfindung war es daher, eine verbesserte Abdichtung der Durchflusszelle zu erreichen bei gleichzeitig guter Reinigbarkeit und Bedienerfreundlichkeit der Zellen.

Gelöst wird die Aufgabe mit einer Durchflusszelle, einem Dissolution Testgerät und einem Verfahren zur Prüfdurchführung mit einem Dissolution Testgerät gemäß den unabhängigen Ansprüchen. Besonders bevorzugte Gestaltungsvarianten der Erfindung finden sich in den abhängigen Ansprüchen.

Erfindungsgemäß wird eine verbesserte Abdichtung dadurch erzielt, dass auf die üblichen O-Ring-Dichtungen sowie eine äußere Klemmvorrichtung für die gesteckten Teile verzichtet wird. Stattdessen wird der zylindrische Zellenmantel mit der Zellenaufnahme und dem Filterkopf jeweils mittels eines Schraubgewindes verbunden. Dies erlaubt eine sichere Führung der Teile bei gleichzeitiger bedienungsfreundlicher Zerlegbarkeit. Da bei der Verschraubung keine elektrische oder mechanische Klemmvorrichtung auf der Oberseite des Zellenkopfes angebracht werden muss, kann die Auslassöffnung für das Prüfmedium, die im Stand der Technik immer seitlich am Kopf angeordnet ist, auf der Oberseite angeordnet werden.

Dies sorgt für eine verbesserte Strömungsführung im Kopfbereich, da die 90°-Umlenkung der Strömung entfällt. Damit wird auch eine gleichmäßigere Filterflächennutzung erzielt. Der Filter wird dazu erfindungsgemäß im hohl ausgeführten, schraubbaren Filterkopf angeordnet. Die bislang üblichen Varianten können entfallen. Der Filter wird einheitlich als Filterpaket im Filterkopf oberhalb der Dichtung untergebracht, was ein bequemeres Einsetzen ermöglicht.

Höchst bevorzugt erfolgt die Abdichtung zwischen der Zellenaufnahme, mit der die Durchflusszelle auf dem Wasserbad befestigt wird, und der Probenzelle über eine stirnseitige Flachdichtung. Da die Einzelteile durch die Verschraubung exakt geführt werden und bedingt durch eine im Vergleich zu den toroiden O-Ring-Dichtungen breitere Ausgestaltung der Flachdichtung wird eine bessere Abdichtung erreicht, die zudem weniger anfällig für Handhabungsfehler des Laborpersonals ist, da ein Verkanten praktisch ausgeschlossen werden kann.
Ferner erfolgt höchst bevorzugt auch die Abdichtung zwischen dem Zellenmantel und der Zellenaufnahme und zwischen dem Zellenmantel und dem Filterkopf jeweils über eine stirnseitige Flachdichtung. Damit kann zuverlässig eine Leckage nach außen sowie innerhalb des Filterkopfs verhindert werden. Besonders letzterer Bereich ist kritisch für die Messungen, da hier die Gefahr einer Vermischung von Wasser und Prüfmedium besteht.

Ein erfindungsgemäßes Dissolution Testgerät verfügt über mindestens eine solche Durchflusszelle. In einer höchst bevorzugten Ausgestaltungsvariante beinhaltet das Dissolution Testgerät sieben erfindungsgemäße Durchflusszellen. Damit lassen sich in einem Durchlauf mit einem einzigen Gerät alle Testvarianten durchführen, die für die Auflösungsprüfungen vorgeschrieben sind.

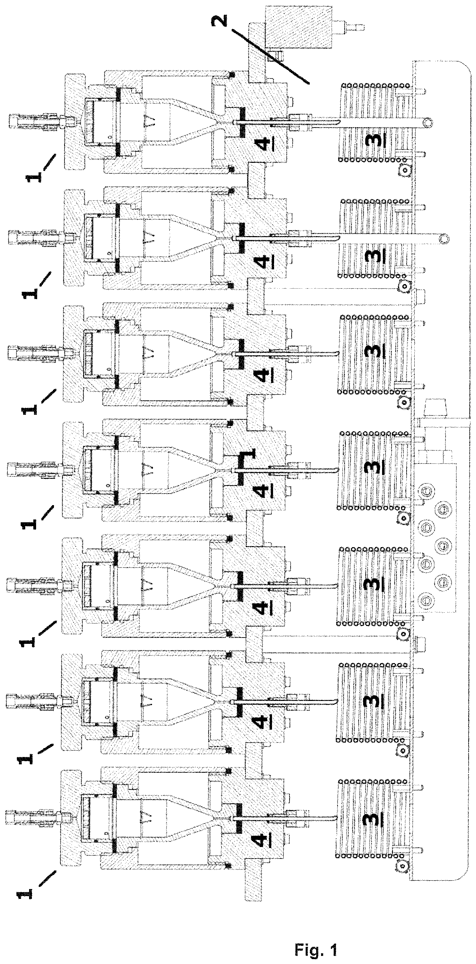

In Fig. 1 ist eine bevorzugte Variante des Dissolution Testgeräts mit sieben Durchflusszellen in einer Schnittdarstellung abgebildet. Die sieben Durchflusszellen (1) sind auf dem Wasserbad (2) des Testgeräts, in das die Heizspiralen (3) der Durchflusszellen (1) eintauchen, mittels ihrer Zellenaufnahmen (4) montiert. Die Zellenaufnahmen (4) werden fest mit dem Oberteil des Wasserbads (2) verschraubt und verbleiben im normalen Betrieb des Geräts auch dort. Zu Wartungszwecken kann die Zellenaufnahme (4) nach Lösen der seitlichen Schrauben jedoch entnommen werden.

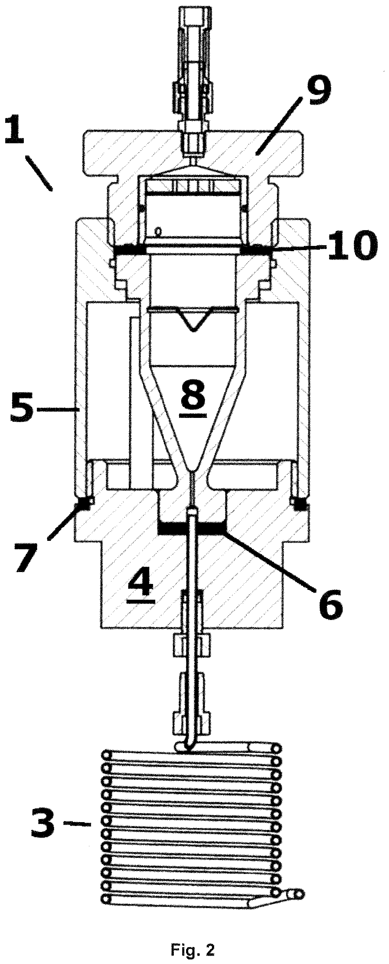

In Fig. 2 ist eine Schnittdarstellung einer einzelnen Durchflusszelle (1) mit ihrer zugehörigen Heizspirale (3) abgebildet. Auf der Zellenaufnahme (4) wird mit einem Innengewinde der Zellenmantel (5) aufgeschraubt. Im Zentrum der Zellenaufnahme (4) ist eine Flachdichtung (6) eingelegt und in einer Rille am äußeren Umfang die Flachdichtung (7). Im Zentrum des Zellenmantels (5) wird die Probenzelle (8) eingesetzt. Dabei sitzt die Unterseite der Probenzelle (8) in der Aussparung der Zellenaufnahme (4) stirnseitig auf der Flachdichtung (6) auf, die eine Öffnung für die Prüfmedium-Zufuhr aufweist. Die Abdichtung des Zellenmantels (5) zur Zellenaufnahme (4) hin wird durch die stirnseitige Flachdichtung (7) erzielt.

Der schraubbare Filterkopf (9), in den ein Filterpaket eingelegt ist, wird von oben mit einem Außengewinde in den Zellenmantel (5) eingeschraubt. Dabei dichtet die stirnseitige Flachdichtung (10) den Filterkopf (9) gegen den Zellenmantel (5) sowie die Probenzelle (8) ab. Der Auslass für das Prüfmedium ist zentral auf der Oberseite des Filterkopfs (9) angeordnet.

Zur Prüfungsdurchführung wird die Probe in die Probenzelle (8) eingebracht. Der Raum um die Probenzelle (8) wird zur Temperierung mit Wasser aus dem Wasserbad durchspült. Das Prüfmedium wird mit konstanter Flussgeschwindigkeit durch die Heizspirale (3) gepumpt und erwärmt sich dabei auf die Wasserbadtemperatur. Danach strömt es durch den Einlass in die Probenzelle (8), umströmt die Probe und verlässt die Probenzelle (8) am oberen Ende durch das Filterpaket und den Auslass.

### Bezugszeichenliste

- 1: Durchflusszelle
- 2: Heizbad
- 3: Heizspirale
- 4: Zellenaufnahme
- 5: Zellenmantel
- 6, 7, 10: Flachdichtung
- 8: Probenzelle
- 9: Filterkopf

## Patentansprüche

1. Durchflusszelle (1) für ein Dissolution Testgerät, beinhaltend
• eine Zellenaufnahme (4), in deren Zentrum eine Flachdichtung (6) und in eine Rille am äußeren Umfang eine Flachdichtung (7) einlegbar ist, und
• einen zylindrischen Zellenmantel (5), in dessen Zentrum eine Probenzelle (8) zur Probenaufnahme eingesetzt ist, wobei der Raum um die Probenzelle (8) zur Temperierung mit Wasser aus dem Wasserbad (2) durchspülbar ist und die Probenzelle (8) einen Einlass für Prüfmedium enthält, so dass die Probe umströmbar ist, und
• einen Filterkopf (9), auf dessen Oberseite am oberen Ende der Probenzelle (8) zentral ein Auslass für Prüfmedium angeordnet ist,
• worin der Zellenmantel (5) mit dem Filterkopf (9) mittels eines Schraubgewindes verbunden ist,
**dadurch gekennzeichnet, dass**
• die Zellenaufnahme fest mit dem Oberteil eines Wasserbads (2) des Dissolution Testgeräts verschraubbar ist und im normalen Betrieb des Geräts auch dort verbleibt, und
• der Zellenmantel (5) mit der Zellenaufnahme (4) mittels eines Schraubgewindes verbunden ist.

2. Durchflusszelle (1) nach Anspruch 1 oder 2, **dadurch gekennzeichnet, dass** die Abdichtung zwischen der Zellenaufnahme (4) und der Probenzelle (8) über eine stirnseitige Flachdichtung (6) erfolgt.

3. Durchflusszelle (1) nach Anspruch 1 oder 2, **dadurch gekennzeichnet, dass** die Abdichtung zwischen dem Zellenmantel (5) und der Zellenaufnahme (4) und zwischen dem Zellenmantel (5) und dem Filterkopf (9) jeweils über eine stirnseitige Flachdichtung (7, 10) erfolgt.

4. Dissolution Testgerät, **dadurch gekennzeichnet, dass** es ein Wasserbad (2) und mindestens eine Durchflusszelle (1) nach einem der Ansprüche 1-3 beinhaltet.

5. Dissolution Testgerät nach Anspruch 4, **dadurch gekennzeichnet, dass** es sieben Durchflusszellen (1) nach einem der Ansprüche 1-3 beinhaltet.

6. Dissolution Testgerät nach Anspruch 5, **dadurch gekennzeichnet, dass** in das Wasserbad (2) des Dissolution-Testgeräts Heizspiralen (3) der Durchflusszellen (1) eintauchen, durch die zur Prüfungsdurchführung Prüfmedium mit konstanter Flussgeschwindigkeit pumpbar ist, wodurch das Prüfmedium auf die Wasserbadtemperatur erwärmbar ist.

7. Verfahren zur Prüfungsdurchführung mit einem Dissolution Testgerät nach Anspruch 6, umfassend die Schritte
• Einbringen einer Probe in die Probenzelle (8) zur Prüfungsdurchführung,
• Durchspülung des Raums um die Probenzelle (8) mit Wasser aus dem Wasserbad (2) zur Temperierung,
worin
• die Durchflusszellen (1) auf dem Wasserbad (2) des Testgeräts, in das die Heizspiralen (3) der Durchflusszellen (1) eintauchen, mittels ihrer Zellenaufnahmen (4) montiert sind, und die Zellenaufnahmen (4) fest mit dem Oberteil des Wasserbads (2) verschraubt werden und im normalen Betriebszustand dort verbleiben, und
• das Prüfmedium durch die Heizspirale (3) mit konstanter Flussgeschwindigkeit, welches sich dadurch auf die Temperatur des Wasserbads (2) erwärmt, gepumpt wird, und
• das Prüfmedium durch den Einlass in die Probenzelle (8) strömt, die Probe umströmt und die Probenzelle (8) am oberen Ende durch ein Filterpaket, das in den schraubbaren Filterkopf (9) eingelegt ist, und den Auslass verlässt.

## Claims

1. A flow cell (1) for a dissolution test device, containing
• a cell receptacle (4), in the centre of which a flat gasket (6) can be inserted and on the outer circumference a flat gasket (7) can be inserted into a groove, and
• a cylindrical cell sheath (5), in the centre of which a sample cell (8) is used for sample collection, wherein the space around sample cell (8) is flushable with water from a water bath (2) to control the temperature, and sample cell (8) contains an inlet for test medium, such that said sample can flow, and
• a filter head (9), on the top of which an outlet for test medium is centrally arranged at the upper end of sample cell (8),
• wherein cell sheath (5) is connected to filter head (9) by means of a screw thread,
**wherein**
• said cell receptacle is firmly screwed to the upper part of a water bath (2) of the dissolution test device and also remains there during normal operation of the device, and
• cell sheath (5) is connected to cell receptacle (4) by means of a screw thread.

2. Flow cell (1) according to Claim 1 or 2, **wherein** the seal between cell receptacle (4) and sample cell (8) is achieved by means of a flat gasket (6) on front side.

3. Flow cell (1) according to Claim 1 or 2, **wherein** the seal between cell sheath (5) and cell receptacle (4) and between cell sheath (5) and filter head (9) is respectively achieved by means of a flat gasket (7, 10) on the front side.

4. A dissolution test device, **wherein** it comprises a water bath (2) and at least one flow cell (1) according to one of the Claims 1-3.

5. Dissolution test device according to Claim 4, **wherein** it comprises seven flow cells (1) according to one of the Claims 1-3.

6. Dissolution test device according to Claim 5, **wherein** heating coils (3) of flow cells (1) submerge into water bath (2) of said dissolution test device, through which test medium can be pumped at a constant flow rate to carry out the test, as a result of which said test medium can be heated to the temperature of the water bath.

7. A method for carrying out the test with a dissolution test device according to Claim 6, comprising the steps
• introduction of a sample into sample cell (8) to carry out the test,
• flushing the space around sample cell (8) with water from water bath (2) to control the temperature,
wherein
• flow cells (1) on water bath (2) of the test device, into which heating coils (3) of flow cells (1) submerge, are mounted by means of their cell receptacles (4), and cell receptacles (4) are screwed firmly to the upper part of water bath (2) and remain there during normal operating mode, and
• said test medium is pumped through heating coil (3) at a constant flow rate, which therefore heats up to the temperature of water bath (2), and
• said test medium flows through the inlet into sample cell (8), flows around said sample and exits sample cell (8) at the upper end through a filter pack, which is inserted into threaded filter head (9), and the outlet.

## Revendications

1. Cellule à circulation (1) pour un testeur de dissolution, comprenant
• une absorption cellulaire (4) dans le centre de laquelle on peut insérer un joint plat (6) et un joint plat (7) dans une rainure sur le pourtour extérieur, et
• un manteau cellulaire cylindrique (5) dans le centre duquel on utilise une cellule échantillon (8) afin de recevoir l'échantillon, tandis que l'espace autour de la cellule échantillon (8) peut être rincé avec de l'eau issue du bain-marie (2) afin de réguler la température et que la cellule échantillon (8) comprend une entrée pour un fluide d'essai afin que l'échantillon puisse être contourné par l'écoulement, et
• une tête de filtre (9) avec une sortie pour fluide d'essai arrangée sur sa partie supérieure à l'extrémité supérieure de la cellule échantillon (8),
• tandis que le manteau cellulaire (5) est fixé à la tête de filtre (9) au moyen d'un filetage,
**caractérisée par le fait que**
• l'absorption cellulaire puisse être fermement vissée à la partie supérieure d'un bain-marie (2) du testeur de dissolution et y reste également lors du fonctionnement normal de l'appareil, et
• le manteau cellulaire (5) soit fixé à l'absorption cellulaire (4) au moyen d'un filetage.

2. Cellule à circulation selon la revendication 1 ou 2, **caractérisée par le fait que** l'étanchéité entre l'absorption cellulaire (4) et la cellule échantillon (8) se fasse via un joint plat sur la face frontale (6).

3. Cellule à circulation selon la revendication 1 ou 2, **caractérisée par le fait que** l'étanchéité entre le manteau cellulaire (5) et l'absorption cellulaire (4) et entre le manteau cellulaire (5) et la tête de filtre (9) se fasse dans chacun des cas via un joint plat sur la face frontale (7,10).

4. Testeur de dissolution **caractérisé par le fait qu'**il contient un bain-marie (2) et au moins une cellule à circulation (1) selon l'une des revendications 1 à 3.

5. Testeur de dissolution selon la revendication 4 **caractérisé par le fait qu'**il contient sept cellules à circulation (1) selon l'une des revendications 1 à 3.

6. Testeur de dissolution selon la revendication 5, **caractérisé par le fait que** des corps de chauffe en spirale (3) des cellules à circulation (1) trempent dans le bain-marie (2) du testeur de dissolution, à travers desquels un fluide d'essai peut être pompé à une vitesse d'écoulement constante pour l'exécution du contrôle, tandis que le fluide d'essai peut être chauffé à la température du bain-marie.

7. Processus d'exécution du contrôle avec un testeur de dissolution selon la revendication 6, comprenant les étapes suivantes :
• Introduction d'un échantillon dans la cellule échantillon (8) pour l'exécution du contrôle,
• Rinçage de l'espace autour de la cellule échantillon (8) avec de l'eau issue du bain-marie (2) afin de réguler la température,
tandis que
• les cellules à circulation (1) sur le bain-marie (2) du testeur, dans lequel les corps de chauffe en spirale (3) des cellules à circulation (1) trempent, sont montées au moyen de leurs absorptions cellulaires (4), et que les absorptions cellulaires (4) sont fermement vissées à la partie supérieure du bain-marie (2) et y restent lors d'un fonctionnement normal, et
• le fluide d'essai est pompé via le corps de chauffe en spirale (3) avec une vitesse d'écoulement constante et se réchauffe donc à la température du bain-marie (2), et
• le fluide d'essai circule dans la cellule échantillon (8) via l'entrée, contourne l'échantillon et quitte la cellule échantillon (8) à l'extrémité supérieure via un bloc filtrant, lequel est inséré dans la tête de filtre à vis (9), ainsi que la sortie.
